# EUROPEAN PATENT APPLICATION

(11) **EP 4 434 455 A1**
(43) Date of publication of application: **25.09.2024**
(21) Application number: 24161643.2
(22) Date of filing: 06.03.2024
(51) Int. Cl.: A61B 5/103, A61B 5/00, A61B 5/11

(54) **COMBINED SMART SENSING PAD**

(30) Priority: 20.03.2023 TW 112110203
(71) Applicant: Decentralized Biotechnology Intelligence Co., Ltd., Taipei City, Da'an Dist. 106021 (TW)
(72) Inventor: CHOU, Yao Sheng, 106021 Taipei (TW); LIN, Hsiao Yi, 106021 Taipei (TW); CHOU, Yen Han, 106021 Taipei (TW)
(74) Representative: Zaboliene, Reda

(57) **Abstract**

A combined smart sensing pad monitoring system, which includes a planar sensing pad array having a plurality of pressure sensing pads, each of them is a wireless pressure sensing device configured to sense the pressure data of different areas of the planar sensing pad array, an external electronic computing device communicatively connected to the planar sensing pad array to receive the pressure data detected by the plurality of pressure sensing pads. The external electronic computing device combines the pressure data detected by the plurality of pressure sensing pads with the positioning information associated with individual pressure sensing pads to form a pressure distribution. The external electronic computing device monitors the pressure distribution and duration to determine whether to issue a warning.

## Description

### TECHNICAL FIELD

The present invention relates to a sensing pad, more specifically, to a combined smart sensing pad.

### BACKGROUND

As society gradually moves toward an aging population, healthcare for the elderly has become serious issues and challenges. According to statistics, many elderly die due to accidental injuries each year, and falls are one of the major incidents causing accidental injuries. Therefore, how to prevent the elderly from falling will become a top priority for young generations.

With the development of science and technology, economic progress is affecting the society and changing people's lifestyles. How to use modern achievements to serve elderly daily life is the driving force for the continuous progress of the technology. The elderly monitor may effectively realize the living conditions of the elderly, and provide assistant to them in a timely manner when abnormalities are discovered.

The traditional elderly monitor is generally carried out by the surveillance cameras at home for real-time detection. However, the detection requires manual real-time on-line monitor. Only by viewing the screen can you find the problems in time when the issues arise. Viewing the video is time consuming task, it is not only high cost, but also inconvenient, the problem is unlikely to be solved at the first time. The efficiency of the conventional monitor is low, even if a problem is discovered, it is impossible to assist the elderly immediately.

Further, the traditional sensing pad is not practical due to one-size facility cannot fit all home environment. The present invention discloses sensing pads, for example, pressure sensing pads, are employed to monitor the activity of elderly, and detect elderly fall caused by accidental collision or physiological state.

### SUMMARY OF THE INVENTION

Based on above, the purpose of the present invention is to provide a combined smart sensing pad comprising a planar sensing pad array including pluralities of pressure sensing pads to sense pressure data in different areas, in one example, the pluralities of pressure sensing pads including a wireless communication device. One of the pluralities of pressure sensing pads is designated to act a master device and remaining others are slave devices, the master device is communicatively coupled with the slave devices to collect pressure data and transmit the pressure data to an external computing device.

In one aspect of the present invention, the master device is set in a master mode, and the slave devices are set in a slave mode. The external computing device is coupled to the planar sensing pad array to receive the pressure data and positioning information to form a pressure distribution. Each one of the pluralities of pressure sensing pads includes a processing device electrically connected to a pressure sensing device for processing pressure data fed by the pressure sensing device; the wireless communication device is electrically connected to the processing device. The pressure sensing device includes at least one pressure sensor.

The processing device includes an application specific integrated circuit (ASIC), a field programmable gate array (FPGA) or a microcontroller. In one embodiment, the processing device and the wireless communication device are integrated to form a sensing circuit. The wireless communication device includes a Bluetooth device. The external computing device determines whether or not to issue a warning based on the pressure distribution, and the external computing device collects pressure point information from the pluralities of pressure sensing pads; wherein the external computing device determine whether number of the pressure point is greater than a threshold, if positive, the external computing device issues a warning signal. In one embodiment, each one of the pluralities of pressure sensing pads includes a light sensor.

In another one aspect of the present invention, a combined smart sensing pad comprises a planar sensing pad array including pluralities of pressure sensing pads to sense pressure data in different areas; wherein each one of the pluralities of pressure sensing pads includes a processing device, a wireless communication device is coupled to the processing device. The pressure sensors are coupled to the processor to collect pressure at different positions, a master/slave mode switch is connected to the processor for setting a mode. The master pad is communicatively coupled with slave pads to collect pressure data and transmit the pressure data to the external computing device, wherein the pluralities of pressure sensing pads are spliced together by a snapping structural.

### BRIEF DESCRIPTION OF THE DRAWINGS

Figure 1A shows a top view of sensing pads according to the present invention.
Figure 1B shows a functional diagram of a sensing pad according to the present invention.
Figure 2 shows monitor system of the sensing pads according to the present invention.
Figure 3A shows a power connecting scheme of a sensing pad array according to the present invention.
Figure 3B shows the power and ground layout according to the present invention.
Figure 4A shows the sensing pad matrix is mapped to the display of the portable device according to the present invention.
Figure 4B shows the functional diagram of the portable device according to the present invention.
Figure 5 shows the monitoring flow chart according to the present invention.
Figure 6 shows an alternative functional diagram of the sensing pad according to the present invention.

### DETAILED DESCRIPTION

Some preferred embodiments of the present invention will now be described in greater detail. However, it should be recognized that the preferred embodiments of the present invention are provided for illustration rather than limiting the present invention. In addition, the present invention can be practiced in a wide range of other embodiments besides those explicitly described, and the scope of the present invention is not expressly limited except as specified in the accompanying claims.

The shape of the conventional pressure sensing pad is standard and one size, it is inconvenience for the elderly people due to home environmental is difference. In order to improve the conventional drawbacks, the present invention discloses a novel sensing pad.

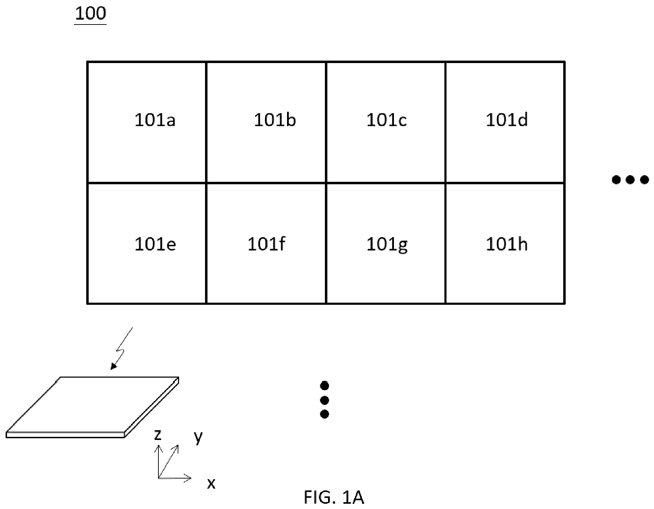

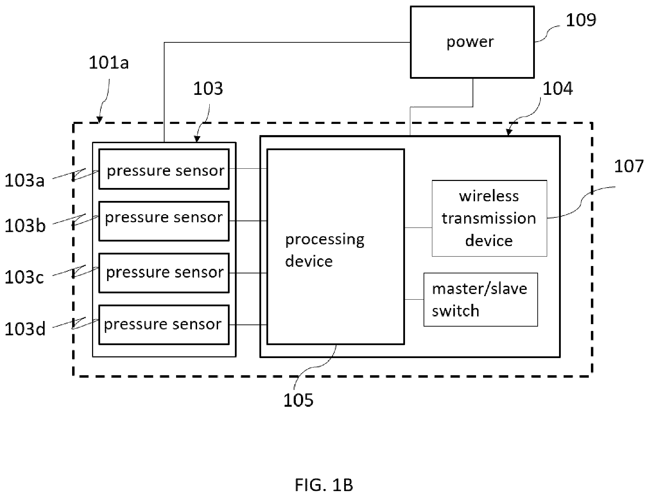

FIG. 1A shows a schematic assembly diagram of a combined smart sensing pad according to an embodiment of the present invention. As shown in Figure 1, each individual one of the sensing pads (101a, 101b, 101c, 101d...) can be spliced with one another to form a planar sensing pad array 100 extending along the X direction and the Y direction.

In one embodiment, the smart sensing pad is a pressure sensing pad (for example, 101a, 101b, 101c, 101d...). The pad structure is a flat body with two main directions (x, y directions) and a thickness direction (z directions). The coordinates (x, y, z) indicate the directions. Pluralities of pressure sensors (not shown) are installed inside the flat body. The flat body is basically square with four sides, but it is not limited to this shape. It could be rectangular as well. The flat body is made of waterproof and anti-slip material. Therefore, it prevents the sensors and circuits from moisture.

In one embodiment, several individual pressure sensing pads could be spliced together along two directions (x, y directions) to form a combined smart sensing pad array by snapping structural on adjacent sides of individual pressure sensing pads. Preferably, buckles or snap devices are formed on four sides of the pads for snapping the sensing pads together. A simplified manner is shown in Fig. 1A and the detailed structure is not shown, the buckles or snap devices are well known in the art.

In one embodiment, the pressure sensor in the pad is a resistive type, a capacitive type or a piezoelectric type.

In one embodiment, the size and the surface area of the individual pressure sensing pads can be changed and adjusted according to the application.

FIG. 1B shows a block diagram of each individual pressure sensing pad, for example, 101a, pressure sensing pad is a wireless pressure detector. The pressure sensing pad 101a includes a pressure sensing device 103 which includes pluralities of pressure sensors (103a, 103b, 103c, 103d) that are electrically connected to the processing device 105 in the sensing circuit 104 by wires to feed the detected pressure signal to the processing device 105. The wireless transmission device 107 receives the pressure signal processed by the processing device 105 and transmits the signal to an external device. The pressure sensing device 103 and the sensing circuit 104 (including the processing unit 105 and the wireless transmission device 104) are configured to receive powered from an external power supply 109. According to the embodiment of the present invention, individual pressure sensing pad, for example pad 101a, is a pressure detector with wireless communication devices and are set to the master mode or the slave mode.

In one embodiment, the wireless communication device 107 is a Bluetooth communication device.

In one embodiment, the processing device 105 includes an application specific integrated circuit (ASIC), a programmable logic circuit (field programmable gate array, FPGA), a microcontroller, etc.

In one embodiment, the sensing circuit 104 and the wireless communication device can be integrated into a single IC chip.

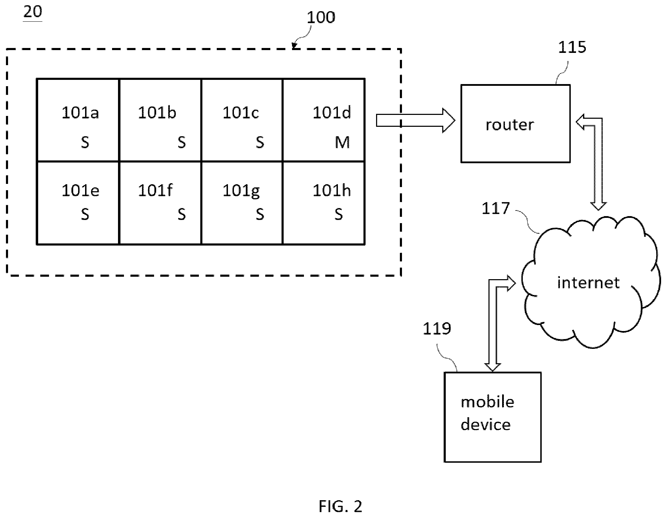

Figure 2 shows a combined smart sensing pad monitoring system 20 including a planar sensing pad array 100 composed of pluralities of pressure sensing pads (101a, 101b, 101c...), a router 115, and an internet 117 and a mobile device 119. One of the pluralities of the pressure sensing pads (101a, 101b, 101c...), for example, the pressure sensing pad 101d is set to play the role of the master device (master mode), and the others are set to act the slave device (slave mode). The master device (pressure sensing pad 101d) is communicatively connected to pluralities of the slave devices (the remaining pressure sensing pads 101a, 101b, 101c, 101e, 101f, 101g, 101h), and the master device (pressure sensing pad 101d) is communicatively connected to other slave devices for collecting pressure data detected by other slave devices (for example, the pressure sensing pads 101a, 101b, 101c, 101e, 101f, 101g, 101h) by wireless transmission, and followed by transmitting the detected data to the mobile device 119 by the router 115 and the internet 117.

In one embodiment, each pressure sensing pad in the planar sensing pad array can be regarded as a pressure point Pi, (i=1, 2, 3, ...), and the pressure point pressure is the average pressure values of all the pressures detected by the pluralities of pressure sensors contained therein.

In one embodiment, the mobile device 119 may be one of the following external electronic computing devices such as a smartphone, a tablet computer, or a notebook computer.

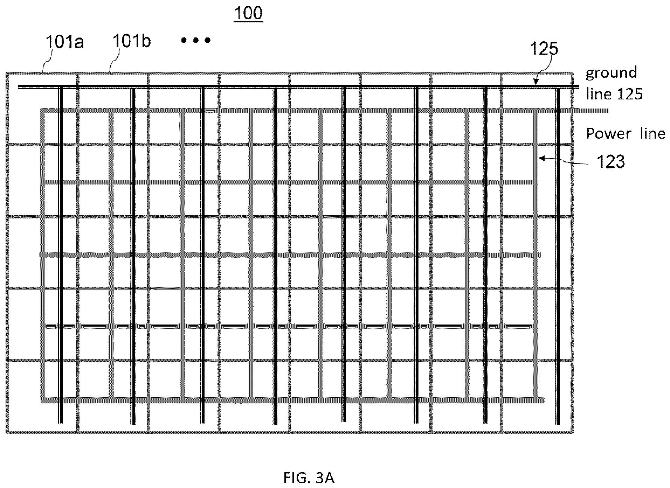

FIG. 3A shows a schematic diagram of the power connections in the planar sensing pad array 100. The diagram shows the wiring configuration of the power lines 123 and the power ground lines 125 provided in the smart sensing pad, wherein the power lines 123 are indicated by bold lines, the ground wires 125 are shown as composite lines.

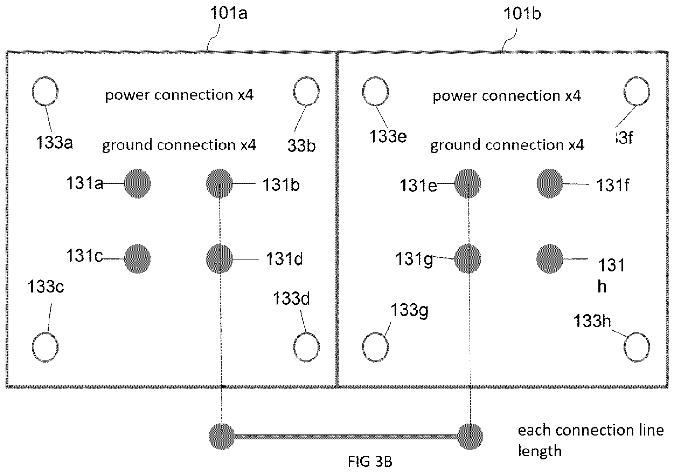

Figure 3B shows a schematic diagram of the configuration of the power and ground connections; each smart sensing pad (101a, 101b) has up to 4 sets of power connection points including power contacts (131a, 131b, 131c, 131d) and ground contacts (133a, 133b, 133c, 133d). The power connection between smart sensing pads (for example, between smart sensing pads (101a, 101b)) is shown in FIG. 3B, it can be achieved by connecting wires across the connection points (131b, 131e).

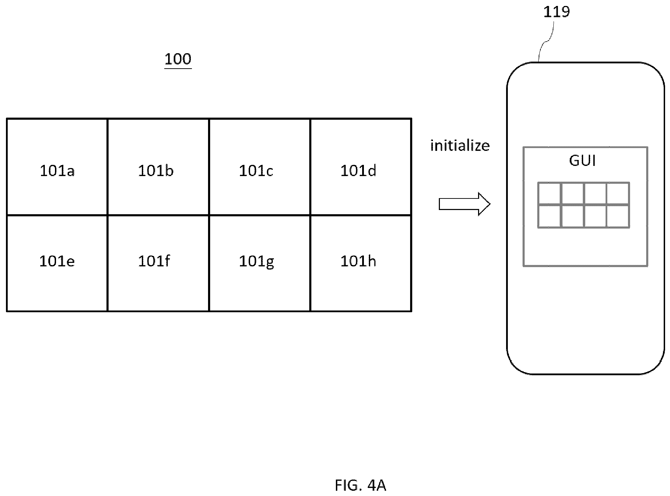

FIG. 4A shows a schematic diagram of mapping position information of the combined planar sensing pad array 100 to the display of the mobile device according to an embodiment of the present invention. After assembling the planar sensing pad array 100, all smart sensing pads 101a to 101h are connected with the master pad, the connection between the master pad and the mobile device 119 is established as well. The mobile device 119 (or the server 121) can perform the configuration operations for mapping the positioning marks (coordinates) of the sensing pads 101a to 101h to a user graphical interface GUI on the display device of the mobile device 119 for monitoring the pressure response distribution of the sensing pad array 100. The mapping method is well-known in the art of mapping technology. The pressure response distribution of the planar sensing pad array 100 refers to pressure distribution for short, it includes the pressure data and position information (sensing pad coordinates), and they can be monitored by the external electronic computing device to determine whether or not to issue a warning signal to a designated person.

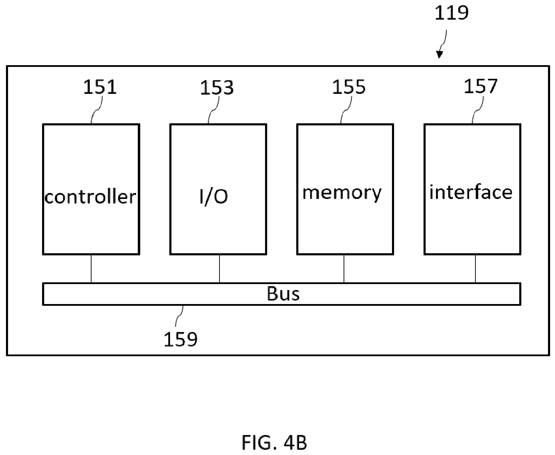

FIG. 4B shows a functional block diagram of the mobile device 119. The mobile device 119 may include a controller 151, an input/output (I/O) device 153, a memory 155 and an interface 157. These components may be connected to each other via bus 159.

The controller 151 may include a microprocessor, a digital signal processor, and any similar processing device. I/O device 153 may include at least one of a keypad and a display. Memory 155 may be used to store instructions executed by the controller. In some embodiments, the interface 157 may be configured to connect to the wireless transmission module 107 of the pressure sensing pad 101a via a wireless network (refer to FIG. 2).

When the mobile device 119 receives a signal (or data) through the wireless network interface, the mobile device 119 can process the signal to generate a pressure distribution corresponding to different locations or positions, and followed by outputting the illustration to the display device (screen). The external electronic computing device combines the pressure data detected by the pressure sensing pads with the position information associated with individual pressure sensing pads to form a pressure distribution.

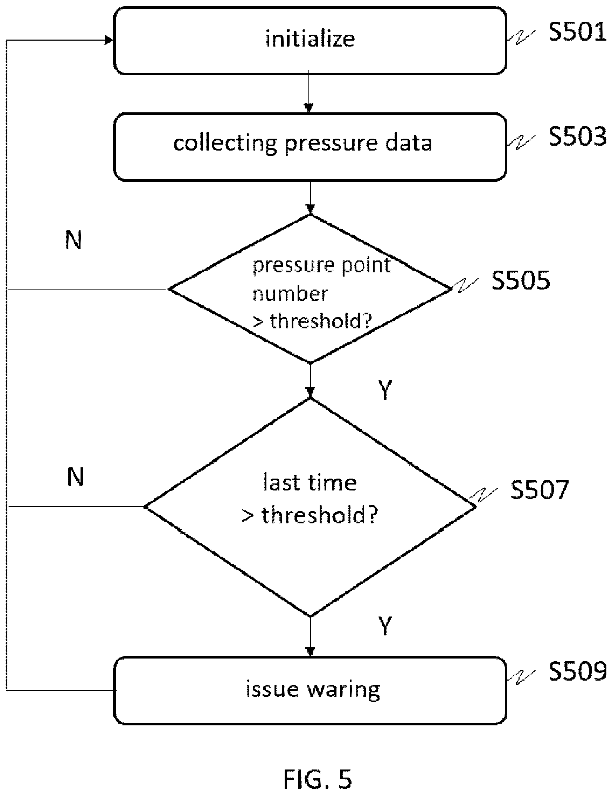

Figure 5 shows a flow chart of the monitoring process executed by the combined smart sensing pad monitoring system. First, step S501 initializes the sensing pads, namely, the planar sensing pad array 100. All smart sensing pads 101a to 101h are connected with the master device, and the connection between the master device and the mobile device 119 is also established. Subsequently, step S503, the sensing pads collect the pressure point position data. One pressure point corresponds to an activated pressure sensing pad, the more the sensing pads activates, the more the pressure points are. Next step S505 is to determine whether the number of pressure points is greater than a threshold or not, if positive, it means that the contact area (or the number of activated pressure pads) is larger than the normal standing area, the elderly may be at risk of falling; at this time, step S507, the system further determines whether the situation continues for more than a preset time period (for example, several minutes). If it is positive, an alarm is sent out by the wireless communication device (step S509) to an alarm receiving device with Bluetooth, WiFi or other wireless communication function, in one example, the alarm receiving device is a smart phone, laptop, tablet computer, etc.

The above description is only an explanation of the fall prevention function. The combined pressure sensing pad monitoring system disclosed by the present invention can also be applied to other occasions, such as (1). as a smart mattress for preventing bedsore monitoring; (2). laying the sensing pads on the balcony as an anti-theft device to remove window bars and beautify the city appearance; (3). laying the sensing pads on indoor space as a falling detection for preventing solitary elderly from lonely deaths.

In addition, according to another aspect of the present invention, each smart pad can also be equipped with a light sensor and transmit the detected signal to the external device through the wireless device such as Bluetooth. For example, if there is someone in the bathroom, the light of the sensing pads should be turned on, and when someone falls, large area of the smart pads and the corresponding light sensors will be shaded.

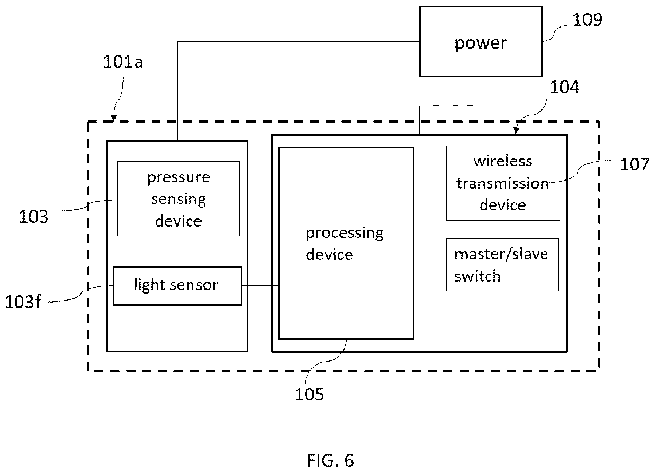

In one embodiment, please refer to Figure 6, it shows that the light sensors are formed in the respective smart pads (pressure sensing pads; similar to Figure 1B). Each pressure sensing pad 101a, in addition to the pressure sensing device 103, it also includes at least one light sensor 103f which is electrically connected to the processing device 105 of the sensing circuit 104. In this embodiment, each sensing pads (101a, 101b, 101c...) not only sense the pressure, but also detect the light. The configuration of the master-slave device mode is identical to the embodiment shown in Figure 2. The planar sensing array can also be employ to detect the light. Similar to the monitoring process in Figure 5, when the shading area of the planar sensing array exceeds a preset threshold, the elderly may be at risk of falling; at this time, the system further determines whether or not the time of shading lasts longer than the pre-set time period (for example, several minutes), if positive, an alarm is sent out by wireless communication.

As will be understood by persons skilled in the art, the foregoing preferred embodiment of the present invention illustrates the present invention rather than limiting the present invention. Having described the invention in connection with a preferred embodiment, modifications will be suggested to those skilled in the art. Thus, the invention is not to be limited to this embodiment, but rather the invention is intended to cover various modifications and similar arrangements included within the spirit and scope of the appended claims, the scope of which should be accorded the broadest interpretation, thereby encompassing all such modifications and similar structures. While the preferred embodiment of the invention has been illustrated and described, it will be appreciated that various changes can be made without departing from the spirit and scope of the invention.

## Claims

1. A combined smart sensing pad comprising:
a planar sensing pad array (100) including pluralities of pressure sensing pads (101a, 101b, 101c, 101d...) to sense pressure data in different areas, each one of said pluralities of pressure sensing pads (101a, 101b, 101c, 101d...) including a wireless communication device (107); and
wherein one of said pluralities of pressure sensing pads (101a, 101b, 101c, 101d...) is a master device and others are slave devices, said master device being coupled with said slave devices to collect pressure data and transmit said pressure data to an external computing device (119).

2. The combined smart sensing pad of claim 1, wherein said external computing device (119) is coupled to said planar sensing pad array (100) to receive said pressure data and positioning information to generate a pressure distribution.

3. The combined smart sensing pad of claim 1, wherein each one of said pluralities of pressure sensing pads (101a, 101b, 101c, 101d...) includes a pressure sensing device (103), a processing device (105) electrically connected to said pressure sensing device (103) for processing said pressure data; said wireless communication device (107) being electrically connected to said processing device (105).

4. The combined smart sensing pad of claim 3, wherein said pressure sensing device (103) includes pluralities of pressure sensors (103a, 103b, 103c, 103d).

5. The combined smart sensing pad of claim 3, wherein said processing device (105) includes an application specific integrated circuit (ASIC), a field programmable gate array (FPGA) or a microcontroller.

6. The combined smart sensing pad of claim 3, wherein said wireless communication device includes (107) a Bluetooth device.

7. The combined smart sensing pad of claim 1, said pluralities of pressure sensing pads (101a, 101b, 101c, 101d...) are spliced together along two directions to form said planar sensing pad array (100).

8. The combined smart sensing pad of claim 1, wherein said external computing device determines whether number of a pressure point is greater than a threshold or not, if positive, said external computing device (119) issuing a warning signal.

9. The combined smart sensing pad of claim 1, wherein each one of said pluralities of pressure sensing pads (101a, 101b, 101c, 101d...) includes a light sensor.

10. The combined smart sensing pad of claim 4, wherein said pressure sensors (103a, 103b, 103c, 103d) include capacitor type pressure sensor.

11. The combined smart sensing pad of claim 4, wherein said pressure sensors (103a, 103b, 103c, 103d) include resistant type pressure sensor.

12. The combined smart sensing pad of claim 4, wherein said pressure sensors (103a, 103b, 103c, 103d) include piezoelectric type pressure sensor.

13. The combined smart sensing pad of claim 1, wherein said pluralities of pressure sensing pads (101a, 101b, 101c, 101d...) are made of waterproof and anti-slip material.

14. The combined smart sensing pad of claim 1, wherein said external computing device (119) performs configuration operations for mapping positioning marks of said pluralities of pressure sensing pads (101a, 101b, 101c, 101d...) to a user graphical interface GUI of said external computing device (119) for monitoring the pressure response distribution of the sensing pad array (100).
